(19) Europäisches Patentamt / European Patent Office / Office européen des brevets

(11) **EP 3 905 880 B1**

(12) **EUROPEAN PATENT SPECIFICATION**

(45) Date of publication and mention
of the grant of the patent:
**12.06.2024 Bulletin 2024/24**

(21) Application number: **19907032.7**

(22) Date of filing: **23.12.2019**

(51) International Patent Classification (IPC):
**A01N 1/02** (2006.01)   **A61K 35/36** (2015.01)
**A61K 35/44** (2015.01)

(52) Cooperative Patent Classification (CPC):
**C12N 5/0068; C12N 7/00;** C12N 2500/32;
C12N 2500/80; C12N 2501/11; C12N 2501/39;
C12N 2710/16151

(86) International application number:
**PCT/US2019/068230**

(87) International publication number:
**WO 2020/142304 (09.07.2020 Gazette 2020/28)**

(54) **SUPPLEMENTED SERUM-CONTAINING CULTURE MEDIUM FOR ENHANCED ARPE-19 GROWTH AND HUMAN CYTOMEGALOVIRUS VACCINE PRODUCTION**

ERGÄNZTES SERUMHALTIGES KULTURMEDIUM ZUR STEIGERUNG DES WACHSTUMS VON ARPE-19 UND ZUR HERSTELLUNG EINES IMPFSTOFFS GEGEN DAS HUMANE CYTOMEGALOVIRUS

MILIEU DE CULTURE CONTENANT DU SÉRUM ENRICHI POUR LA CROISSANCE D'ARPE -19 ACTIVÉ ET LA PRODUCTION D'UN VACCIN CONTRE LE CYTOMÉGALOVIRUS HUMAIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.01.2019 US 201962787929 P**

(43) Date of publication of application:
**10.11.2021 Bulletin 2021/45**

(73) Proprietor: **Merck Sharp & Dohme LLC**
**Rahway, New Jersey 07065 (US)**

(72) Inventors:
• **CHRISTANTI, Sianny**
  **West Point, Pennsylvania 19486 (US)**
• **LIM, Esther Wei Yee**
  **La Jolla, California 92037 (US)**
• **RODRIGUEZ, Jason**
  **Watertown, Massachusetts 02472 (US)**
• **DANIELS, Christopher, L.**
  **West Point, Pennsylvania 19486 (US)**

(74) Representative: **Merck Sharp & Dohme LLC**
**120 Moorgate**
**London EC2M 6UR (GB)**

(56) References cited:
WO-A1-2011/009294      WO-A1-2013/071151
CN-A- 108 913 646      US-A1- 2013 149 284
US-A1- 2014 178 987      US-B2- 7 932 084

• **TATSUMA YAO ET AL: "Animal-cell culture media: History, characteristics, and current issues", REPRODUCTIVE MEDICINE AND BIOLOGY, vol. 16, no. 2, 1 April 2017 (2017-04-01), pages 99-117, XP055703756, Tokyo ISSN: 1445-5781, DOI: 10.1002/rmb2.12024**
• **NAKAMURA TSUYOSHI ET AL: "Optimization of cell line development in the GS-CHO expression system using a high-throughput, single cell-based clone selection system", JOURNAL OF BIOSCIENCE AND BIOENGINEERING, vol. 120, no. 3, 1 September 2015 (2015-09-01), pages 323-329, XP055951005, NL ISSN: 1389-1723, DOI: 10.1016/j.jbiosc.2015.01.002**

- **PRISTOVSEK NUSA ET AL: "Using Titer and Titer Normalized to Confluence Are Complementary Strategies for Obtaining Chinese Hamster Ovary Cell Lines with High Volumetric Productivity of Etanercept", BIOTECHNOLOGY JOURNAL, vol. 13, no. 3, 9 February 2018 (2018-02-09), page 1700216, XP055951006, DE ISSN: 1860-6768, DOI: 10.1002/biot.201700216 Retrieved from the Internet: URL:https://api.wiley.com/onlinelibrary/td m/v1/articles/10.1002%2Fbiot.201700216>**
- **BELHAJ: "Enhancements in Alginate Microencapsulation Technology & Impacts on Cell Therapy Development", Doctoral dissertation, May 2018 (2018-05), XP055660732, Retrieved from the Internet: URL:https://scholarcommons.sc.edu/cgi/view content.cgi?article=5489&context=etd [retrieved on 2020-02-21]**

Remarks:

The complete document including Reference Table(s) and the Sequence Listing(s) can be downloaded from the EPO website

Remarks:

**Description**

FIELD OF INVENTION

The present invention relates to supplemented serum-containing cell culture media that provides enhanced ARPE-19 cell growth and/or improved yields of human cytomegalovirus (HCMV) grown in ARPE-19 cell cultures. The culture media of the invention includes a glucocorticoid hormone such as dexamethasone, a growth factor such as epidermal growth factor (EGF), additional essential amino acids, nucleosides (e.g., as provided in glutamine synthetase expression medium supplement) and various trace elements. The invention further provides methods of producing HCMV in such culture media.

BACKGROUND OF THE INVENTION

Human cytomegalovirus (HCMV), also known as human herpesvirus 5 (HHV-5), is a herpes virus classified as being a member of the beta subfamily of herpesviridae. HCMV is an enveloped virus, having an outer lipid bilayer envelope containing a variety of glycoproteins including glycoprotein B (gB), gH, gL, gM, gN, and gO, with a double stranded linear DNA core in an icosahedral nucleocapsid. See Crough et al., 2009, Clinical Microbiology Reviews 22:76-98.

According to the Centers for Disease Control and Prevention, HCMV infection is found fairly ubiquitously in the human population, with an estimated 40-80% of the United States adult population having been infected. The virus is spread primarily through bodily fluids and is frequently passed from pregnant mothers to the fetus or newborn. In most individuals, HCMV infection is latent, although virus activation can result in high fever, chills, fatigue, headaches, nausea, and splenomegaly. HCMV infections in immunocompromised individuals (such as HIV-positive patients, allogeneic transplant patients and cancer patients) or persons whose immune system has yet to be fully developed (such as newborns) can be particularly problematic. See Mocarski et al., 2007, Cytomegalovirus, In Knipes and Howley (Eds.), Field Virology pp. 2701-2772. HCMV infection in such individuals can cause severe morbidity, including pneumonia, hepatitis, encephalitis, colitis, uveitis, retinitis, blindness, and neuropathy, among other deleterious conditions. In addition, HCMV infection during pregnancy is a leading cause of birth defects. See, *e.g.,* Adler, 2008, J. Clin Virol 41:231; Arvin et al., 2004, Clin Infect Dis 39:233; and Revello et al., 2008, J Med Virol, 80:1415.

To date, there is no available HCMV vaccine. However, a promising candidate based on a live attenuated HCMV is currently in clinical trials. See U.S. Pat. No. 9,546,355 and Wang et al., 2016, Sci Transl Med 8:362ra145. This vaccine is a genetically modified HCMV produced using human retinal pigment epithelial cells (ARPE-19).

For commercial vaccine production based on a live virus, optimization of the production process, including cell culture and its culture media, is critical. Despite potential problems associated with serum-containing culture media for HCMV production, HCMV production in serum-free culture media has failed to provide adequate virus yield. Thus, to date, serum-containing culture media is required for HCMV production.

Serum-free culture media containing a combination of dexamethasone and EGF for the preparation of vaccines has been described in U.S. Pat. No. 6,656,719 (rotavirus vaccine) and Japanese Patent Application Publication No. JP2006158388 (polio vaccine).

Serum-containing media containing glucocorticoids such as dexamethasone have shown mixed results in viral production and infectivity. See Costa et al., 1974, Nature 252:745-746; Solodushko et al., 2009, Am J Physiol Lung Cell Mol Physio 297:L538-45; Tanaka et al., 1984, J Gen Virol. 65:1759-67; and Tanaka et al., 1984, Virology 136:448-52. Treatment of cells with pharmacological concentrations of adrenal glucocorticoids such as dexamethasone enhanced HCMV replication; treatment with oestrogenic or androgenic hormones did not do so.

SUMMARY OF THE INVENTION

The present invention relates to supplemented serum-containing cell culture media that provides enhanced ARPE-19 cell growth and/or improved yields of human cytomegalovirus (HCMV) grown in ARPE-19 cell cultures. The culture media of the invention includes a glucocorticoid hormone such as dexamethasone, a growth factor such as epidermal growth factor (EGF), additional essential amino acids, nucleosides (e.g., as provided in glutamine synthetase expression medium supplement) and various trace elements.

In certain embodiments, the invention provides a cell culture medium comprising a basal cell culture medium that comprises serum and is further supplemented with between about 0.1 $\mu$M and about 10 $\mu$M of dexamethasone, between about 0.1 ng/mL and about 100 ng/mL of epidermal growth factor (EGF), 1X MEM essential amino acids, 1X-4X glutamine synthetase expression medium (GSEM) supplement and trace elements. In one aspect, the basal cell culture medium is DMEM/F-12. In certain aspects, the serum is fetal bovine serum (FBS), optionally present at a concentration between 2% and 10%, inclusive.

In certain embodiments, the dexamethasone is present at a concentration between about 1 and about 10 μM. In certain embodiments, the EGF is present at a concentration between about 1 and about 40 ng/mL. In certain embodiments, the 1X MEM essential amino acids consist of arginine, cysteine, histidine, isoleucine, leucine, lysine, methionine, phenylalanine, threonine, tryptophan, tyrosine, and valine. In certain embodiments, the 1X GSEM supplement consists of a mixture of L-alanine, L-asparagine, L-aspartic acid, L-glutamic acid, L-proline, L-serine, adenosine, guanosine, cytidine, uridine, and thymidine. In certain embodiments, the trace elements are selected from cupric sulfate, ferric citrate, sodium selenite, and zinc sulfate.

The certain embodiments, the invention provides a composition comprising a cell culture medium of the invention and cells selected from ARPE-19, Vero, MDCK, MRC-5, BHK, CEM, and LL-I cells. In certain aspects of this embodiment, the composition further comprises a virus. In certain sub-aspects of this aspect, the virus is a human herpesvirus, optionally a human cytomegalovirus (HCMV). The HCMV can be a conditional replication defective HCMV mutant or a recombinant genetically modified HCMV. The conditional replication defective HCMV can have the genomic sequence of SEQ ID NO: 1.

The present invention is also directed to a method of increasing virus production in cells, said method comprising culturing any of the compositions described herein comprising a virus, wherein said composition provides increased production of the virus by at least 10% relative to virus grown in a composition lacking the supplements described above.

BRIEF DESCRIPTION OF THE DRAWINGS

Figure 1 demonstrates statistical analysis assessing the impact of supplements on cell growth.

Figure 2 includes a graph demonstrating the effect of supplements on viral infectivity.

Figure 3 includes a graph demonstrating the difference between supplemented and non-supplemented media.

Figure 4 includes a graph demonstrating culture passage of various media.

Figure 5A-5C include graphs that demonstration improved ARPE-19 cell growth with addition of select supplements at various sera types

Figure 6 includes a graph that compares the volumetric production of virus as measured by Apogee flow cytometry.

DETAILED DESCRIPTION OF THE INVENTION

The present invention is directed to the production of HCMV in ARPE-19 cell culture with supplemented serum-containing culture media. The present invention is based on the discovery that supplementation of culture media with dexamethasone, EGF, essential amino acids, nucleosides and various trace elements to a basal DMEM/F-12 culture medium containing serum reduces the ARPE-19 doubling time as well as increases HCMV virus particles by up to 37% for ARPE-19 cells in static cultures and on microcarriers. The effect of these supplements results in increased cell density per growth vessel for a given time and/or increased productivity of virus on a per-cell basis. The culture media of the invention can be used to increase the production efficiency of HCMV, including conditional replication-defective strains that can be used in prophylactic vaccines.

While dexamethasone and EGF have been used as supplements for virus propagation in serum-free media, the additional of these supplements provided comparable virus yields to serum-containing media. When dexamethasone has been used in serum-containing culture medium, results have been conflicting. For example, HSV-2 viral productivity decreased in SME (Swiss mouse embryo) cells, but increased in NIH3T3 cells, and retroviral infectivity was increased in rat pulmonary microvascular endothelial cells. See Costa et al., 1974, Nature 252:745-746; and Solodushko et al., 2009, Am J Physiol Lung Cell Mol Physio 297:L538-45. Dexamethasone has also been shown to inhibit foot-and-mouth disease virus (FMDV) in infected cattle. See Ilott et al., 1997, Epidemiol Infect 118:181-7. Applicants have found that the addition of dexamethasone and EGF (as well as MEM essential amino acids, GSEM supplement and trace elements) to serum-containing media improves the yield of HCMV in ARPE-19 cells.

The present invention was first demonstrated in micro-scale culture (96-well plates) and T-flasks, and later extended to microcarrers. The compositions and methods of the invention can also be used in bioreactors for large-scale virus production (e.g., for commercial viral vaccine production). The process is scalable from laboratory scale (<3L) to manufacturing scales (*e.g.,* 50 - 2000L) and capable of ultizing a range of feed streams, including medium from static cell culture (*e.g.,* from cell stacks) and culture in bioreactors.

Applicants believe this is the first time that this particular combination of dexamethasone, EGF, essential amino acids, nucleosides and trace elements has been used for viral propagation in serum-containing media to enhance cell yield and virus productivity.

As used herein, purification of "Human cytomegalovirus" or "HCMV" generally refers to purification of HCMV virus particles.

As used herein, "cell culture medium" or "culture medium" refers to the liquid portion of a cell culture having one or more components other than HCMV particles, such as, but not limited to, proteins, nucleic acids, lipids, various cell culture medium components and additives. HCMV particles are released by the cells into the cell culture medium without any intervention or the cells may be lysed or rendered permeable to virus through chemical agents or mechanical means. The cell culture medium can be partially purified using centrifugation, clarification or other methods. In certain embodiments, the cell culture medium contains HCMV through its release into the cell culture.

**Cell Culture Media**

The invention provides serum-containing cell culture media that is supplemented with a glucocorticoid hormone such as dexamethasone, a growth factor such as EGF, additional essential amino acids, nucleosides (from glutamine synthetasae expression medium supplement), and a trace element mixture and is capable of increasing ARPE-19 cell growth and/or the viral yield in the production of HCMV. These supplements can be added to any basal cell culture medium suitable for the culture of cells, particularly ARPE-19 cells, which support virus growth. Exemplary basal culture media include OptiPro™ SFM (ThermoFisher Scientific), VP-SFM AGT™ (ThermoFisher Scientific), SFM4MegaVir (GE Healthcare), Roswell Park Memorial Institute medium (RPMI) 1640 medium, Minimal Essential Medium (MEM), Dulbecco's modified Eagle medium (DMEM), DMEM/F-12 (ThermoFisher Scientific), Eagle's MEM (EMEM), and any other medium in which the cells and virus can grow, as can be tested using standard methods.

The basal cell culture medium is formulated with serum (e.g., fetal bovine serum (FBS)), which is standard for most cell culture applications and provides additional nutrients. Generally, serum will be added at about 1% to about 15%. Suitable serum-containing culture media includes, but is not limited to, DMEM/F12 medium supplemented with about 2% to about 10% fetal bovine serum (FBS), DMEM supplemented with about 2% to about 10% FBS, and EMEM (Eagle's Minimum Essential Medium) supplemented with about 1% to about 15% FBS.

Generally, the basal culture medium will contain inorganic salts for regulation of the osmolality, preferably in the range of 200-400 milliOsmoles (mOss), and more preferably in the range of 290-350 mOsm. Osmolality regulators are generally salts. Inorganic salts used in the basal medium of the present invention include, for example, salts of $Ca^{2+}$, $K^+$, $Mg^{2-}$, $Na^+$, $CO_3^{2-}$, $PO_4^{3-}$. Any salt of each of these moieties can be present in the basal cell culture media. For example, the following salts can be used: $CaCh$, $KCl$, $KNO_3$, $MgSO_4$, $NaCl$, $NaHCO_3$, and $NaH_2PO_4 \cdot 2H_2O$.

The basal cell culture medium will also contain buffering agents, which act to stabilize the hydrogen ion concentration and therefore the pH of a solution by neutralizing, within limits, both acids and bases. Buffering agents commonly used in the cell cloning medium maintain the pH in the range about 6.5-7.5, preferably about pH 7.0, and include carbonates such as $NaHCO_3$; chlorides, sulphates and phosphates such as $CaCl_2 \cdot 2H_2O$, $MgSO_4 \cdot 7H_2O$, $NaH_2PO_4 \cdot 2H_2O$, $Na_2HPO_4 \cdot 7H_2O$, beta-glycerol-phosphate, bicarbonate or sodium pyruvate. Such buffers are generally present in an amount from about 50-3000 mg/liter. Other buffers, such as N-[2-hydroxyethyl]piperazine-N'-[2-ethanesulphonic acid] otherwise known as HEPES and 3-[N-Morpholino]-propanesul-fonic acid otherwise known as MOPS can be present in an amount from about 1000-10,000 mg/liter.

The basal cell culture medium will further contain an energy source, such as a carbohydrate. The energy source of use in the cell cloning medium is preferably a monosaccharide, such as mannose, fructose, galactose or maltose, preferably glucose, and particularly D-glucose, which is generally present in an amount from about 1000-10,000 mg/liter.

Vitamins and enzyme co-factor vitamins (co-factors), such as Vitamin B6 (pyridoxine), Vitamin B 12 (cyanocobalamin) and Vitamin K (biotin) may be present in the basal cell culture medium, preferably in an amount of about 0.01-0.5 mg/liter. Vitamin C (ascorbic acid) may be present in an amount of about 10-30 mg/liter, and Vitamin B2 (riboflavin) may be present in an amount of about 0.1-1.0 mg/liter. The basal cell culture medium may also contain Vitamin B1 (thiamine), nicotine amide, Vitamin B5 (D calcium pentothenate), folic acid, and/or i-inositol, which are preferably present in an amount of about 0.2-8.0 mg/liter.

Basal cell culture medium will also typically include a lipid agent, which provides a source of lipids or contributes to lipid formation. Suitable lipid agents which can be used in the basal cell culture medium include, but are not limited to, Human Ex-Cyte® (Bayer), ethanolamine (or a salt thereof), sitosterol (a plant steroid), rice hydrolysate (a mixture of proteins and lipids), LTI Defined Lipid Mixture, a mixture of arachidonic acid, cholesterol, DL-alpha-tocopherol-acetate, ethyl alcohol, linoleic acid, linolenic acid, myristic acid, oleic acid, palmitric acid, palmitic acid, Pluronic® F-68, stearic acid, Tween® 80, choline chloride, phosphatidylcholine and methyl lineoleate. Preferably, ethanolamine is used in basal cell culture medium. Suitable concentrations of a lipid agent ingredient can be determined by one of ordinary skill in the art. Lipid agents will generally be present in an amount of about 0.05-10 mg/liter.

The components of DMEM/F12 media are provided in Table 1.

Table 1: Components of DMEM/F 12

| Components | Concentration (mg/L) | mM |
|---|---|---|
| **Amino Acids** | | |
| Glycine | 18.75 | 0.25 |
| L-Alanine | 4.45 | 0.05 |
| L-Arginine hydrochloride | 147.5 | 0.7 |
| L-Asparagine-$H_2O$ | 7.5 | 0.05 |
| L-Aspartic acid | 6.65 | 0.05 |
| L-Cysteine hydrochloride-$H_2O$ | 17.56 | 0.1 |
| L-Cystine 2HCl | 31.29 | 0.1 |
| L-Glutamic Acid | 7.35 | 0.05 |
| L-Glutamine | 365.0 | 2.5 |
| L-Histidine hydrochloride-$H_2O$ | 31.48 | 0.15 |
| L-Isoleucine | 54.47 | 0.416 |
| L-Leucine | 59.05 | 0.451 |
| L-Lysine hydrochloride | 91.25 | 0.499 |
| L-Methionine | 17.24 | 0.116 |
| L-Phenylalanine | 35.48 | 0.215 |
| L-Proline | 17.25 | 0.15 |
| L-Serine | 26.25 | 0.25 |
| L-Threonine | 53.45 | 0.449 |
| L-Tryptophan | 9.02 | 0.044 |
| L-Tyrosine disodium salt | 55.79 | 0.214 |
| L-Valine | 52.85 | 0.452 |
| **Vitamins** | | |
| Biotin | 0.0035 | 1.434E-5 |
| Choline chloride | 8.98 | 0.064 |
| D-Calcium pantothenate | 2.24 | 0.005 |
| Folic Acid | 2.65 | 0.006 |
| Niacinamide | 2.02 | 0.017 |
| Pyridoxine hydrochloride | 2.013 | 0.001 |
| Riboflavin | 0.219 | 5.82E-4 |
| Thiamine hydrochloride | 2.17 | 0.006 |
| **Components** | **Concentration (mg/L)** | **mM** |
| Vitamin B12 | 0.68 | 5.018E-4 |
| i-Inositol | 12.6 | 0.07 |
| **Inorganic Salts** | | |
| Calcium Chloride ($CaCl_2$) | 116.6 | 1.050 |
| Cupric sulfate ($CuSO_4$-$5H_2O$) | 0.0013 | 5.2E-6 |

(continued)

| Inorganic Salts | | |
|---|---|---|
| Ferric Nitrate | 0.05 | 1.24E-4 |
| Ferric sulfate (FeSO$_4$-7H$_2$O) | 0.417 | 0.002 |
| Magnesium Chloride | 28.64 | 0.302 |
| Magnesium Sulfate (MgSO$_4$) | 48.84 | 0.407 |
| Potassium Chloride (KCl) | 311.8 | 4.157 |
| Sodium Bicarbonate (NaHCO$_3$) | 2438.0 | 29.024 |
| Sodium Chloride (NaCl) | 6995.5 | 120.612 |
| Sodium Phosphate dibasic | 71.02 | 0.500 |
| Sodium Phosphate monobasic | 62.5 | 0.453 |
| Zinc sulfate (ZnSO$_4$-7H$_2$O) | 0.432 | 0.002 |
| **Other Components** | | |
| D-Glucose (Dextrose) | 3151.0 | 17.506 |
| Hypoxanthine Na | 2.39 | 0.015 |
| Linoleic Acid | 0.042 | 1.5E-4 |
| Lipoic Acid | 0.105 | 5.1E-4 |
| Phenol Red | 8.1 | 0.022 |
| Putrescine 2HCl | 0.081 | 5.031E-4 |
| **Components** | **Concentration (mg/L)** | **mM** |
| Sodium Pyruvate | 55.0 | 0.5 |
| Thymidine | 0.365 | 0.002 |

The components of FBS are provided in Table 2. Due to the nature of the purification from animal sources, there can be a significant variance between different samples.

Table 2: Composition of 100% FBS

| Component | Average | Range |
|---|---|---|
| Endotoxins (ng/ml) | 0.35 | 0.01 - 10.0 |
| Glucose (mg/ml) | 1.25 | 0.85 - 1.81 |
| Protein (mg/ml) | 38 | 32 - 70 |
| Albumin (mg/ml) | 23 | 20 - 36 |
| Hemoglobin ($\mu$g/ml) | 113 | 24 - 181 |
| Bilirubin, total ($\mu$g/ml) | 4 | 3 - 11 |
| Bilirubin, direct ($\mu$g/ml) | 2 | 0 - 5 |
| Urea ($\mu$g/ml) | 160 | 140 - 200 |
| Urate ($\mu$g/ml) | 29 | 13 - 41 |
| Creatine ($\mu$g/ml) | 31 | 16 - 43 |
| Insulin ($\mu$U/ml) | 10 | 6 - 14 |
| Cortisol (ng/ml) | 0.5 | 0.1 - 23 |
| Growth hormone (ng/ml) | 39.0 | 18.7 - 51.6 |

(continued)

| Component | Average | Range |
|---|---|---|
| Parathormone, PTH (ng/ml) | 1.72 | 0.085 - 6.18 |
| Triiodothyronine, T3 (ng/ml) | 1.2 | 0.56 - 2.23 |
| Thyroxine, T4 (ng/ml) | 0.12 | 0.08 - 0.16 |
| Thyroid-stimulating hormone, TSH (ng/ml) | 1.22 | 0.2 - 4.5 |
| Follicle-stimulating hormone, FSH (pg/ml) | 95 | 20 - 338 |
| Testosterone (pg/ml) | 400 | 210 - 990 |
| Progesterone, P4 (pg/ml) | 80 | 3 - 360 |
| Prolactin = Luteotropic hormone, LTH (pg/ml) | 176 | 20 - 500 |
| Luteinizing hormone, LH (pg/ml) | 8 | 1,2 - 18 |
| Prostaglandin E (ng/ml) | 5.9 | 0.5 - 30.5 |
| Prostaglandin F (ng/ml) | 12.3 | 3.8 - 42.0 |
| Vitamin A (ng/ml) | 90 | 10 - 350 |
| Vitamin E (ng/ml) | 1.1 | 1 - 4.2 |
| Cholesterol ($\mu$g/ml) | 310 | 120 - 630 |
| Lactate-dehydrogenase, LDH (mU/ml) | 864 | 260 - 1,215 |
| Alkaline Phosphatase (mU/ml) | 255 | 110 - 352 |
| Aspartate-Aminotransferase, ASAT (mU/ml) | 130 | 20 - 200 |
| Sodium, Na+ ($\mu$eq/ml) | 137 | 125 - 143 |
| Potassium, K+ ($\mu$eq/ml) | 11.2 | 10.0 - 14.0 |
| Calcium, Ca2+ ($\mu$eq/ml) | 6.75 | 6.30 - 7.15 |
| Chloride, Cl- ($\mu$eq/ml) | 103 | 98 - 108 |
| Phosphate, Pi ($\mu$g/ml) | 98 | 43 - 114 |
| Selenium ($\mu$g/ml) | 0.026 | 0.014 - 0.038 |
| pH | 7.40 | 7.20 - 7.60 |

* from Lindl, T. (2002): "Zell- und Gewebekultur". 5th ed. Spektrum Akademischer Verlag, Heidelberg

Glucocorticoid compounds include, but are not limited to, betamethasone, dexamethasone, fludrocortisone acetate hydrocortisone, methylprednisolone, prednisolone, prednisone, and triamcinolone. The compounds are readily available from commercial sources such as Sigma-Aldrich, St. Louis, MO. In the medium of the invention the glucocorticoid compound is dexamethasone.

The glucocorticoid compound can be added to a concentration of about 1 nM-1 mM. Preferably glucocorticoid compound is added to a concentration of about 0.1 $\mu$M to about 100 $\mu$M, about 0.5 $\mu$M to about 10 $\mu$M, about 1 $\mu$M to about 5 $\mu$M.

While the glucocorticoid compound can be added to the culture medium prior to addition of cells, i.e., is a component of the cell culture medium, glucocorticoid can be added at inoculation or at any time after virus infection, e.g., the glucocorticoid compound can be added within one day of the virus infection or any time later. In this scenario, the glucocorticoid compound may be added to the cell culture one time, two times, three times, or any number of times during the specified time period. One or more glucocorticoid compounds may be used in conjunction. That is, any given single addition of a glucocorticoid compound may include the addition of one or more other glucocorticoid compounds. Similarly, if there is more than one addition of a glucocorticoid compound, different glucocorticoid compounds may be added at the different additions. Additional compounds and substances, including glucocorticoid compounds, may be added to the culture before, with or after the addition of glucocorticoid compound-either during or not during the specified time period.

The glucocorticoid compound may be added to the cell culture by any means. Means of adding glucocorticoid compound include, but are not limited to, dissolved in DMSO, dissolved in organic solvent such as ethanol, dissolved in water, dissolved in culture medium, dissolved in feed medium, dissolved in a suitable medium, in the form in which it is obtained or any combination thereof.

The growth factor can be selected from an EGF, a platelet derived growth factor (PDGF), and a fibroblast growth factor (FGF). The growth factor may be any animal or mammalian growth factor, such as human, bovine, equine, murine, porcine, Chinese hamster, chicken or rat. Preferably, the growth factor is a human growth factor.

In the medium of the invention, the growth factor is an EGF. The EGF family includes, for example, EGF, TGF-$\alpha$ (transforming growth factor-$\alpha$), amphiregulin, HB-EGF (heparin-binding EGF-like growth factor), epiregulin, and neuregulin. In one aspect of this embodiment, the EGF family member is EGF. The EGF used in the presently claimed medium is preferably of synthetic or recombinant origin. EGF is available from various commercial sources, including Sigma-Aldrich.

The growth factor can be used in concentration of about 0.5 ng/ml to about 400 ng/ml, about 2 ng/ml to about 200 ng/ml, or about 5 ng/ml to about 40 ng/ml.

Accordingly, in certain embodiments, the cell culture medium contains a basal cell culture medium suitable for culturing primary human cells, which basal cell culture medium is preferably enriched and additionally contains: about 0.5 to about 200 ng/ml of EGF and about about 0.5 $\mu$M to about about 10 $\mu$M of dexamethasone. In certain embodiments, the cell culture medium contains about 2.0 to about 50 ng/ml of EGF and about 1 $\mu$M to about 10 $\mu$M of dexamethasone.

The culture media will also contain a mixture of amino acids sufficient to support growth of the cells. The amino acids can be in the basal cell culture media and supplemented as necessary to obtain the concentrations in Table 3.

Table 3: Amino acid concentrations

| Amino Acid | Desired final concentration (mg/liter) |
| --- | --- |
| L-Alanine | 5-50 |
| L-Arginine (HCl) | 50-100 |
| L-Asparagine (H$_2$O) | 5-50 |
| L-Aspartic Acid | 5-50 |
| L-Cystine (disodium salt) | 50-500 |
| L-Glutamic acid | 5-50 |
| L-Glutamine | 100-600 |
| Glycine | 20-200 |
| L-Histidine (HCl.H$_2$O) | 50-500 |
| L-Isoleucine | 50-500 |
| L-Leucine | 50-500 |
| L-Lysine (HCl) | 100-500 |
| L-Methionine | 20-200 |
| L-Phenylalanine | 50-250 |
| L-Proline | 10-100 |
| L-Serine | 20-200 |
| L-Threonine | 50-500 |
| L-Tryptophan | 10-100 |
| L-Tyrosine (disodium salt) | 50-500 |
| L-Valine | 50-500 |

The amino acids included in the medium are preferably of synthetic origin. The amounts which are usually included vary for each amino acid but are generally in the range about 10-500 mg/ml. However, L-glutamine is generally present at much higher concentration preferably in the range about 100-600 mg/ml.

In the medium of the invention, a commercially available amino acids mixture is added to the basal culture media. Representative amino acid mixtures are available as essential amino acids (e.g., MEM (Minimal Essential Medium) Amino Acids Solution (50X), Corning Inc., Corning, NY, MilliporeSigma, or ThermoFisher Scientific).

Nucleosides are included in the cell culture medium of the present invention. Examples of nucleosides include adenosine, cytidine, guanosine, thymidine, and uridine. Each of these nucleosides can be found in the commercially available glutamine synthetase expression medium supplement (e.g., GSEM Supplement (50X), Millipore Sigma, St. Louis, MO). Typically, essential amino acid solutions do not contain L-glutamine. In the medium of the invention the basal culture media is supplemented with both MEM essential amino acids and GSEM.

The composition of MEM and GSEM are provided in Tables 4 and 5, respectively.

Table 4: 1X MEM Essential Amino Acids

| Components | mg/ml | mM |
|---|---|---|
| L-Arginine hydrochloride | 126.4 | 0.5991 |
| L-Cystine | 24.0 | 0.1 |
| L-Histidine hydrochloride-$H_2O$ | 42.0 | 0.2 |
| L-Isoleucine | 52.4 | 0.4 |
| L-Leucine | 52.4 | 0.4 |
| L-Lysine hydrochloride | 72.5 | 0.3962 |
| L-Methionine | 15.1 | 0.1013 |
| L-Phenylalanine | 33.0 | 0.2 |
| L-Threonine | 47.6 | 0.4 |
| L-Tryptophan | 10.2 | 0.05 |
| L-Tyrosine | 36.0 | 0.1989 |
| L-Valine | 46.8 | 0.4 |

The concentrations refer to the amino acids being added to the basal cell culture medium. In cases where a 50X solution is diluted 50-fold to obtain a 1X solution, the above concentrations do not take into account any amino acids already in the culture media. If MEM is used as the basal culture medium, addition of 1X MEM Essential Amino Acids to the MEM medium will result in a 2X concentration of the essential amino acids.

Table 5: 1x GSEM Supplements

| Component | Concentration (mg/L) |
|---|---|
| L-alanine | 9 |
| L-asparagine · $H_2O$ | 85.22 |
| L-aspartic acid | 13 |
| L-glutamic acid | 75 |
| L-proline | 11.5 |
| L-serine | 10 |
| adenosine | 7 |
| guanosine | 7 |
| cytidine | 7 |
| uridine | 7 |
| thymidine | 0.24 |

The concentrations refer to the supplements being added to the basal cell culture medium. In cases where a 50X solution is diluted 50-fold to obtain a 1X solution, the above concentrations do not take into account any of the listed amino acids or nucleosides already in the basal culture media. GSEM Supplement is described in Doyle et al., Cell and Tissue Culture: Laboratory Procedures 27D, 3-3, (1999)

A basal culture medium will also be supplemented with various trace elements, which will be present in a cell culture medium in only trace amounts. Trace elements for supplementation include, for example, $Se^{4+}$, $Ag^+$, $Al^+$, $Ba^{2+}$, $Cd^{2+}$, $Co^{2+}$, $Cr^+$, $Ge^{4+}$, $Br^-$, $I^-$, $Mn^{2+}$, $F^-$, $Si^{4+}$, $V^{5+}$, $Mo^{6+}$, $Ni^{2+}$, $Rb^+$, $Sn^{2+}$ and $Zr^{4+}$ and salts thereof. Particularly common trace elements include non-ferous metal ions of magnesium, copper and zinc; as well as sodium, potassium and selenium. The ions are generally added to the medium in the form of salts such as chlorides and sulphates. Any salt of a given trace element can be used to supplement the basal culture medium. For example, the sodium salt of selenium oxide

(sodium selenite, $Na_2SeO_3$) is preferably used to provide selenium. Suitable concentrations of trace element-containing compounds can be determined by one of ordinary skill in the art. For example, in the medium of the invention, the concentration of $SeO_3^{2-}$ can be about 0.007 to about 0.07 mg/L. In a preferred embodiment of the medium of the invention, the concentration of $SeO_3^{2-}$ is about 0.01 mg/L. The amounts are typically similar to those provided in the media disclosed in the examples below, but may be varied. A representative commercially available mix (Corning™ Trace Elements A, 1000X) when diluted to 1X adds 1.6 $\mu$g/mL $CuSO_4 \cdot 5H_2O$, 863 $\mu$g/mL $ZnSO_4 \cdot 7H_2O$, 17.3 $\mu$g/mL Selenite $\cdot$2Na, 1155.1 $\mu$g/mL ferric citrate. Concentrations of the trace elements in the culture media can be 1x - 4x concentrations.

### Cell Lines

A suitable cell line includes one that hosts HCMV. Suitable host cells include, but are not limited to, epithelial cells (*e.g.* ARPE-19 human retinal pigment epithelial cells), endothelial cells (*e.g.* TIME, HUVEC), fibroblast cells (*e.g.* MRC-5 human fetal lung fibroblast cells, WI-38 human fetal lung fibroblasts, HFF, etc.) and kidney cells (e.g., Vero African green monkey kidney and BHK baby hamster kidney). Typically, the cell line is a mammalian cell line, such as a rodent, non-human primate (e.g., monkey), or human cell line. In one embodiment, the host cell line is ARPE-19.

### Culture conditions

Cell culture is performed to enable the cell to metabolize, grow, divide and/or produce virus of interest. Cell culture conditions suitable for the methods of the present invention are those that are typically employed and known for batch, fed-batch, or continuous culturing of cells, with attention paid to pH, e.g., about 6.5 to about 7.5; dissolved oxygen ($O_2$), e.g., between about 5-90% of air saturation and carbon dioxide ($CO_2$), agitation and humidity, and temperature.

Cell culture methods may comprise growth adhering to surfaces, growth in suspension, or combinations thereof. Culturing can be done, for instance, in dishes, well plates, T-flasks, shake flasks, stirred vessels, spinner flasks, roller bottles or in bioreactors, using batch, fed-batch, continuous systems, hollow fiber, and the like. High density cell culture systems, such as Corning HyperFlask® and HyperStack® systems can also be used. In one embodiment, a suitable cell culturing vessel for large scale virus production is a bioreactor. Bioreactor processes and systems have been developed to optimize gas exchange, to supply sufficient oxygen to sustain cell growth and productivity, and to remove $CO_2$. Maintaining the efficiency of gas exchange is an important criterion for ensuring successful scale up of cell culture and protein production. Such systems are well-known to the person having skill in the art.

The culture may be run for any length of time and may be determined by one of skill in the art, based on relevant factors such as the quantity and quality of recoverable virus, and the level of contaminating cellular species (e.g. proteins and DNA) in the supernatant, which will complicate recovery of the virus of interest. Suitable conditions for culturing cells are known. See, *e.g.*, Tissue Culture, (1973), Kruse and Paterson (Eds.), Academic Press, and Freshney, 2000, Culture of animal cells: A manual of basic technique, fourth edition (Wiley-Liss Inc.).

Methods of propagating cytomegalovirus, including HCMV, are known in the art. See, *e.g.*, Britt, 2010, Human Cytomegalovirus: Propagation, Quantification, and Storage; in Current Protocols in Microbiology, 18:E:14E.3:14E.3.1-14E.3.17; Chambers et al., 1971, Appl Microbiol 22:914-8; and Schneider-Ohrum et al., 2016, J Virol 90:10133-10144. In some embodiments, cell cultures are inoculated with cytomegalovirus and cultured for a period of time (*e.g.*, 24 hours to 3 weeks) before harvesting the virus. Various methods of harvesting virus from cell culture are known in the art. In some embodiments, the cytomegalovirus is harvested from the cell culture by collecting the medium. In some embodiments, the cytomegalovirus is harvested from the cell culture by lysing the cells (*e.g.*, by mechanical or non-mechanical lysis methods). In some embodiments, the cytomegalovirus is harvested from the cell culture by adding a cell permeation agent to release virus from host cells.

In order to achieve large-scale production of virus through cell culture, it is preferred in the art to have cells capable of growing in suspension or microcarriers. Accordingly, in some embodiments, the cells used to propagate the HCMV are grown on microcarriers. A microcarrier is a support matrix allowing for the growth of adherent cells in spinner flasks or bioreactors (such as stirred bioreactors, rotating wall microgravity bioreactors and fluidized bed bioreactors). Microcarriers are typically 125 - 250 $\mu$M spheres with a density that allows them to be maintained in suspension with gentle stirring. Microcarriers can be made from a number of different materials including, but not limited to, DEAE-dextran, glass, polystyrene plastic, acrylamide, and collagen. The microcarriers can have different surface chemistries including, but not limited to, extracellular matrix proteins, recombinant proteins, peptides and charged molecules. In one embodiment, the microcarriers are Cytodex™ microcarriers (GE Healthcare Life Sciences) which are based on cross-linked dextran matrices.

In some embodiments, the processes described herein employ a nuclease to remove contaminating nucleic acids, which are mostly from the host cell. Exemplary nucleases suitable for use in the invention include BENZONASE®, PULMOZYME®, or any other DNase and/or RNase commonly used within the art. In preferred embodiments of the invention, the nuclease is BENZONASE®, which rapidly hydrolyzes nucleic acids by hydrolyzing internal phosphodiester bonds between specific nucleotides. BENZONASE® is a genetically engineered variant of an endonuclease normally expressed in *Serratia marcescens,* and can be commercially obtained from Millipore Sigma (Burlington, MA). The concentration at which the nuclease is employed is preferably within the range of 1-100 units/ml.

In certain embodiments, the nuclease is added to the post-harvest cell culture medium. The batch can then be incubated at temperatures ranging from the culturing temperature (*e.g.*, about 37°C), or at room temperature (around 20°C) or lower (*e.g.*, around 0°C), wherein, in general, longer incubation times are required at lower temperatures to achieve the same result.

In certain embodiments, the nuclease can be employed before the cell culture medium is harvested. It may be added just seconds prior to (or virtually concomitant with) the harvesting step, but preferably, the nuclease is added to the culture at least one minute before the harvesting step and up to several days before the harvesting step. The cell culture with the added nuclease can then be incubated above process temperature, *e.g.*, around 40°C, or at the culturing temperature (*e.g.*, about 37°C), or at room temperature (around 20°C) or lower (*e.g.*, around 0°C), wherein, in general, longer incubation times are required at lower temperatures to achieve the same result.

## Cytomegalovirus

The cell culture media of the present invention can be used to produce any cytomegalovirus, including wild-type cytomegalovirus, a recombinant cytomegalovirus, and modified cytomegalovirus (for example, cytomegalovirus containing a transgene). In some embodiments, the cytomegalovirus is a human cytomegalovirus (HHV-5). In some embodiments, the HCMV is AD169 strain, Toledo strain or Towne strain. In some embodiments, the cytomegalovirus is a recombinant HCMV, particularly, a conditional replication defective HCMV. In some embodiments, the cytomegalovirus is a recombinant HCMV delivering one or more transgenes. The processes of the invention can also be used to purify both infectious and noninfectious HCMV particles. See Schneider-Ohrum et al., 2016, J Virol. 90:10133-10144.

In certain embodiments, the HCMV is a recombinant HCMV vaccine. See, *e.g.,* Lilja et al., 2012, Vaccine 30:6980. In certain embodiments, the HCMV is a recombinant vaccine vector expressing a heterologous antigen. The antigen can be from another pathogen or be a tumor antigen. See, *e.g.,* U.S. Pat. No. 9,249427 (pathogen antigen or tumor antigen), U.S. Patent Application Publication No. 20160354461 (HIV, SIV or TB antigens) and International Patent Application Publication No. WO1998/010311A1 (HIV or malarial antigens); Marzi et al., 2016, Sci Rep 6:21674 (Ebola virus); and Tierney et al., 2012, Vaccine 30:3047 (tetanus toxin).

In one embodiment, the HCMV is a conditional replication defective HCMV (rdHCMV). As used herein, the term "conditional replication defective virus" refers to virus particles that can replicate in a certain environments but not others. In certain embodiments, a virus is made a conditional replication defective virus by destabilization of one or more proteins essential for viral replication such as IE1/2, UL51, UL52, UL79 and UL84, or a combination thereof, for example, by fusion with a destabilizing polypeptide such as a sequence of FKBP or a derivative thereof. Preferred FKBP derivatives have one or more of the following substitutions at the denoted amino acid positions F15S, V24A, H25R, F36V, E60G, M66T, R71G, D100G, D100N, E102G, K105I and L106P. The FKBP derivative having the F36V and L106P substitutions is particularly preferred. The nucleic acids encoding the wild type, nondestabilized essential proteins are modified in the conditional replication defective virus. In more preferred embodiments, one or more essential proteins are destabilized. Such fusion proteins can be stabilized by the presence of a stabilizing agent such as Shield-1, commercially available from Cheminpharma LLC (Farmington, CT) or Clontech Laboratories, Inc. (Mountain View, CA). See, *e.g.,* U.S. Patent Application Publication No. 2009/0215169; and Clackson et al., 1998, Proc Natl Acad Sci USA 95:10437-42).

In one embodiment, the conditional replication defective HCMV is derived from AD 169 strain that has a restored gH complex expression due to a repair of a mutation in the UL131 gene. As used herein, the terms "pentameric gH complex" or "gH complex" refer to a complex of five viral proteins on the surface of the HCMV virion. The complex is made up of proteins encoded by UL128, UL130, and UL131 assembled onto a gH/gL scaffold. See Wang and Shenk, 2005, Proc Natl Acad Sci USA 102:1815; and Ryckman et al., 2008, J. Virol. 82:60.

In one embodiment, the genome of the rdHCMV has a destabilized IE1/2 and UL51 and has a sequence as shown in SEQ ID NO:1. See U.S. Pat. No. 9,546,355.

## Manufacture of Conditional Replication Defective HCMV

rdHCMV can be propagated in the presence of a stabilizing agent such as Shield-1 on permissive cell types, preferably human epithelial cells, and more preferably human retinal pigmented epithelial cells. In additional embodiments, the human retinal pigmented epithelial cells are ARPE-19 cells deposited with the American Type Culture Collection (ATCC) as Accession No. CRL-2302. In some embodiments, Shield-1 is present at a concentration of at least 0.5 $\mu$M in the tissue culture media. In preferred embodiments, Shield-1 is present at a concentration of at least 2.0 $\mu$M in the tissue culture media. Shield-1 can be used to control replication of the rdHCMV in conjunction with FKBP (FK506 binding protein). See, *e.g.,* U.S. Pat. No. 9,546,355.

In certain embodimetns, Shield-1 is one of the components to be removed during the purification steps described herein. After purification of rdHCMV from cell culture media containing Shield-1, there may be a small amount of residual Shield-1 remaining in the rdHCMV preparation. In one embodiment, the level of Shield-1 in the HCMV composition after purification is at least 10-fold below the level used to sustain replication in tissue culture. In another embodiment, the level of Shield-1 in the rdHCMV preparation after purification is 0.05 $\mu$M or less. In another embodiment, the level of Shield-1 in the rdHCMV preparation after purification is undetectable.

Determination of Shield-1 levels in a composition can be detected using a LC/MS (liquid chromatography-mass spectroscopy) or HPLC/MS (high performance liquid chromatography-mass spectroscopy) assays. These techniques combine the physical separation capabilities of LC or HPLC with the mass analysis capabilities of and can detect chemicals of interest in complex mixtures.

**Compositions Including Media, Cells, and, Optionally, Viruses**

The invention also includes compositions that include a medium of the invention, in combination with cells for use in virus production. These compositions can also, optionally, include viruses produced in the cells of the invention. Thus, in a certain embodiment, the invention provides a cell culture media as described herein, in combination with cells, e.g., ARPE-19 cells. In one aspect of the embodiment, the invention provides a cell culture media as described herein, in combination with APRE-19 cells infected with HCMV. The HCMV can include attenuating and/or other beneficial mutations as described herein.

**Isolation of HCMV**

Following cell culture, HCMV is isolated from the cell culture. Typically, cytomegalovirus is released from mammalian host cells into the culture media without any intervention. However, in some cases, cytomegalovirus can by released from cells by by adding a cell permeation agent to release virus from host cells or by lysing host cells. HCMV obtained from culture media, whether released by cells or through cell lysis, will contain one or more contaminants selected from proteins (*e.g.*, host cell proteins, cell culture serum proteins or exogenously added proteins), and nucleic acids *(e.g.,* host cell DNA). The cytomegalovirus preparations can by purified by at least one filtration, chromatography, clarification, precipitation, or fractionation step or any combination thereof. A representative purification process is provided in U.S. Provisional Application No. 62/661,928, filed on April 24, 2018.

An exemplary complete purification process involves: **(1)** Nuclease digestion (with Benzonase®) prior to harvest in a bioreactor containing cell culture medium and microcarrier beads having cells infected with HCMV; **(2)** harvest of cell culture medium from microcarrier beads; **(3)** clarification of harvested cell culture medium by a 1.2-micron glass-fiber filter (*e.g.* Sartorius Sartopore® GF Plus); **(4)** capture of viral particles by anion exchange (AEX) membrane chromatography (*e.g.*, Sartorius Sartobind® Q); **(5)** polishing purification by Capto™ Core 700 chromatography (GE Healthcare) operated in a flow through mode; **(6)** concentration and buffer exchange by 750-kDa hollow fiber TFF *(e.g.,* GE Healthcare or Spectrum). In one aspect, a sterile filtration using a 0.2-micron filter selected from Sartorius Sartobran® P, Millipore Durapore™ PVDF filter, or Pall SuporLife® is performed before or after the TFF step in **(6).** In an alternative aspect, no sterile filtration is performed and the process is run completely aseptically.

In one embodiment, a 750-kDa hollow fiber TFF (GE Healthcare or Spectrum) step may also be included between the AEX and polishing chromatography.

Examples are provided below to further illustrate different features of the present invention. The examples also illustrate useful methodology for practicing the invention. These examples do not limit the claimed invention.

EXAMPLES

ANALYTICAL METHODS

*Viral particle concentration*

A relative size distribution and total concentration of viral particles in the HCMV sample can be obtained by flow cytometry (*e.g.* Apogee Flow Systems). As the individual particles pass through the instrument's flow cell, they are illuminated by a 405 nm laser and the scattered light is detected. The obtained distribution is a relative size distribution of light scattering signal on a logarithmic scale. Counts in total particles per milliliter were also determined.

*Relative infectivity assay*

This analytical procedure is a cell-based relative infectivity assay based on expression of a HCMV protein following infection. ARPE-19 cells were planted and incubated in 96-well micro-titer plates followed by infection with serial dilutions of HCMV samples, controls, and standards. The infected cells were incubated for approximately 1 to 5 days then fixed with a fixative (*e.g.* formaldehyde). After washing, the plate is incubated with a monoclonal antibody (MAb) reactive with a HCMV protein. After incubation with the anti-HCMV MAb, the plate is washed and incubated with a detection antibody. After this final incubation, the plate is washed and read on a plate reader instrument. The relative infectivity assigned to the test article is based on signal generated with an assay reference standard tested on the same plate.

EXAMPLE 1: Screening of individual supplements on ARPE-19 cell growth at 96-well plate format

Despite the preference to avoid animal serum for vaccine production, the propagation of HCMV in ARPE-19 cells requires Fetal Bovine Serum (FBS) in order to obtain sufficient quantities. FBS contains various growth factors, fatty acids, proteins, trace metals, amino acids, etc; some of which are crucial to support mammalian cell growth and virus production within the cell. For serum-free media, supplementation of the media with serum components such as growth factors has been shown to be necessary to achieve levels of virus production similar to that obtained with serum-containing media. In an attempt to optimized virus production in serum-containing media, various supplements were investigated in FBS-containing medium for the purpose of boosting ARPE-19 cell growth and/or CMV virus production without increasing the FBS concentration. This investigation of the effect of supplements on ARPE-19 cell growth was performed on 96-well format, T-flask format and microcarrier inside bioreactors.

The supplements evaluated for their effect on ARPE-19 cell growth include: Epidermal Growth Factor (EGF), Hydrocortisone, Dexamethasone, Insulin, Non-Essential Amino Acids [Glycine, L-Alanine, L-Asparagine, L-Aspartic acid, L-Glutamic Acid, L-Proline, and L-Serine], Essential Amino Acids [L-Arginine, L-Cystine, L-Histidine, L-Isoleucine, L-Leucine, L-Lysine, L-Methionine, L-Phenylalanine, L-Threonine, L-Tryptophan, L-Tyrosine, and L-Valine] (Vendors: MilliporeSigma, ThermoFisher)0, Glutathione, and Linoleic Acid-Oleic Acid-Albumin (Sigma-Aldrich Corp., St. Louis, MO). Several concentrations and combinations were tested as indicated in Table 6

Table 6: Supplements used for screening

| Media | EGF | Hydrocortisone | Dexamethasone | Insulin | Non-ess AA | Glutathione | L+O | Ess. AA | FBS |
|---|---|---|---|---|---|---|---|---|---|
| 1 | 10 | 10 | 0 | 10 | 0 | 0.02 | 1 | 0 | 2% |
| 2 | 10 | 10 | 1 | 0 | 0 | 0.02 | 0 | 1 | 2% |
| 3 | 10 | 10 | 0 | 0 | 1 | 0.02 | 1 | 0 | 2% |
| 4 | 10 | 0 | 0 | 10 | 1 | 0.02 | 0 | 1 | 2% |
| 5 | 0 | 0 | 0 | 0 | 1 | 0.02 | 0 | 0 | 2% |
| 6 | 10 | 0 | 1 | 10 | 1 | 0 | 1 | 0 | 2% |
| 7 | 0 | 10 | 1 | 0 | 1 | 0 | 0 | 0 | 2% |
| 8 | 0 | 10 | 0 | 10 | 0 | 0 | 1 | 1 | 2% |
| 9 | 0 | 10 | 1 | 10 | 1 | 0.02 | 1 | 1 | 2% |
| 10 | 10 | 10 | 0 | 10 | 1 | 0 | 0 | 1 | 2% |
| 11 | 0 | 0 | 1 | 10 | 0 | 0.02 | 0 | 0 | 2% |
| 12 | 0 | 0 | 0 | 0 | 0 | 0 | 1 | 1 | 2% |
| 13 | 10 | 10 | 0 | 10 | 0 | 0 | 0 | 0 | 2% |
| 14 | 10 | 0 | 1 | 0 | 0 | 0 | 1 | 1 | 2% |

Cells were seeded at 1E4 vc/cm$^2$ (viable cells/cm$^2$) and growth was monitored by staining the cells on day 2, 3, 4, 5, 6, 7, and 8 post plant with cell nuclear stain (Hoechst) and quantifying the nuclear stain on an imager to generate cell growth data for each day. The cell growth data was analyzed statistically to assess the impact of the supplement on cell growth.

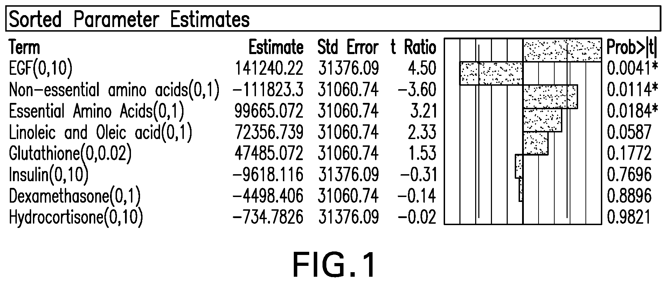

Components with p-value of $< 0.05$ were deemed to have positive effect on cell growth. These include EGF, essential amino acid, linoleic and oleic acid. See Figure 1.

To confirm that these supplements also had an effect on viral infectivity, combinations of multiple supplements at various concentrations were tested in the 96-well plate. ARPE-19 cells were planted at 1E4 viable cell/cm$^2$ in DMEM/F12 containing either 10% FBS or 2% FBS with or without supplements. The cells were allowed to grow for 4 days and growth was monitored by staining the cells on day 2, 3, 4, 5, 6, 7, and 8 post plant with cell nuclear stain (Hoechst) and quantifying the nuclear stain on an imager to generate cell growth data for each day. The cultures were then exchanged into production medium containing 2% FBS with or without supplements and infected with HCMV virus at the multiplicity of infection (MOI) of 0.1 pfu/cell. Culture samples were collected 3 and 4 days post infection and analyzed for virus infectivity (% Response).

| Media | EGF (ng/mL) | Essential Amino Acid (X) | Linoeic + Oleic Acid (X) | Dexamethasone (μM) | Basal Medium during growth | Basal Medium during production |
|---|---|---|---|---|---|---|
| 1 | 10 | | | | DMEM/F12+2%FBS+the supplements specified in the columns | DMEM/F12+2%FBS+the supplements specified in the columns |
| 2 | 50 | | | | DMEM/F12+2%FBS+the supplements specified in the columns | DMEM/F12+2%FBS+the supplements specified in the columns |
| 3 | 100 | | | | DMEM/F12+2%FBS+the supplements specified in the columns | DMEM/F12+2%FBS+the supplements specified in the columns |
| 4 | | 1 | | | DMEM/F12+2%FBS+the supplements specified in the columns | DMEM/F12+2%FBS+the supplements specified in the columns |
| 5 | | 2 | | | DMEM/F12+2%FBS+the supplements specified in the columns | DMEM/F12+2%FBS+the supplements specified in the columns |
| 6 | | 4 | | | DMEM/F12+2%FBS+the supplements specified in the columns | DMEM/F12+2%FBS+the supplements specified in the columns |
| 7 | | | 1 | | DMEM/F12+2%FBS+the supplements specified in the columns | DMEM/F12+2%FBS+the supplements specified in the columns |
| 8 | | | 2.5 | | DMEM/F12+2%FBS+the supplements specified in the columns | DMEM/F12+2%FBS+the supplements specified in the columns |
| 9 | | | 5 | | DMEM/F12+2%FBS+the supplements specified in the columns | DMEM/F12+2%FBS+the supplements specified in the columns |
| 10 | | | | 2.5 | DMEM/F12+2°/ FBS+the supplements specified in the columns | DM E M/F12+2°/ FBS+the supplements specified in the columns |
| 11 | | | | 20 | DMEM/F12+2%FBS+the supplements specified in the columns | DMEM/F12+2%FBS+the supplements specified in the columns |
| 12 | | | | 160 | DMEM/F12+2%FBS+the supplements specified in the columns | DMEM/F12+2%FBS+the supplements specified in the columns |

(continued)

| Media | EGF (ng/mL) | Essential Amino Acid (X) | Linoeic + Oleic Acid (X) | Dexamethasone (μM) | Basal Medium during growth | Basal Medium during production |
|---|---|---|---|---|---|---|
| 13 | 10 | 1 | 1 | 2.5 | DMEM/F12+2%FBS+the supplements specified in the columns | DMEM/F12+2%FBS+the supplements specified in the columns |
| 14 | 50 | 2 | 2.5 | 20 | DMEM/F12+2%FBS+the supplements specified in the columns | DMEM/F12+2%FBS+the supplements specified in the columns |
| 15 | 100 | 4 | 5 | 160 | DMEM/F12+2%FBS+the supplements specified in the columns | DMEM/F12+2%FBS+the supplements specified in the columns |
| 16 | 10 | 1 | | 2.5 | DMEM/F12+2%FBS+the supplements specified in the columns | DMEM/F12+2%FBS+the supplements specified in the columns |
| 17 | | | | | DMEM/F12 with 10% FBS | DMEM/F12+2%FBS |
| 18 | | | | | DMEM/F12 with 2% FBS | DMEM/F12+2%FBS |

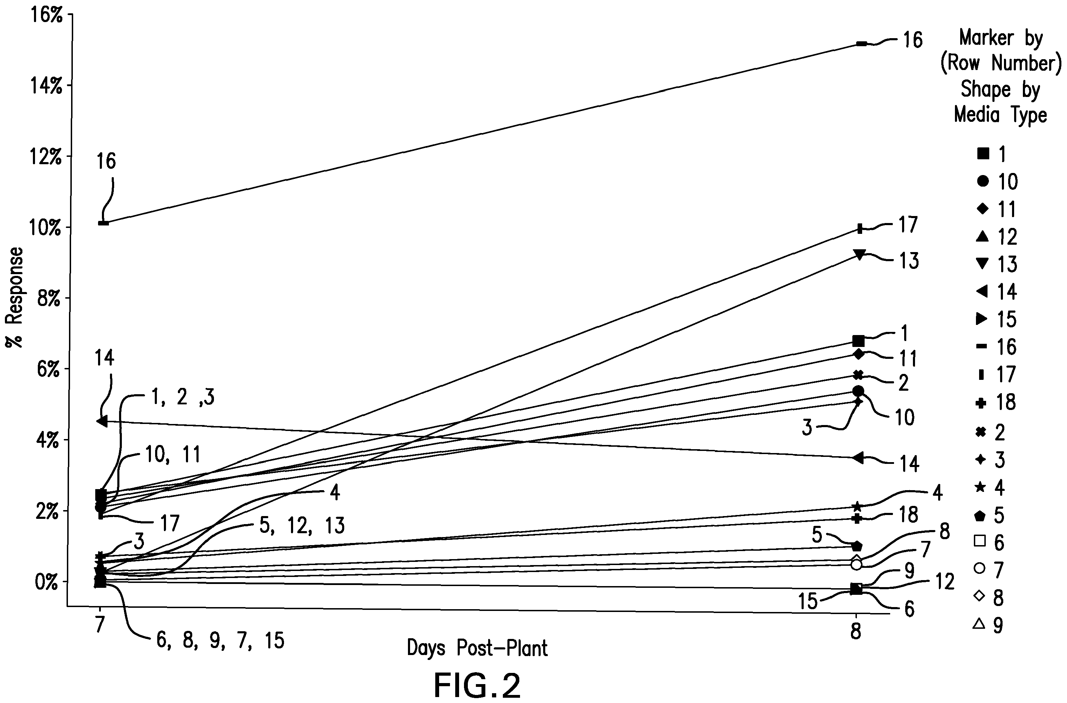

The viral infective was plotted in Figure 2.

The data shown suggests that Media #16 (DMEM/F12+2%FBS+ 10 ng/mL EGF + 1x essential amino acid + 2.5 mcM Dexamethasone) produced the highest virus infectivity compared to other combinations as well as control without supplements.

EXAMPLE 2: Demonstration of the effect of select supplements on ARPE-19 cell growth in T-flasks

An ARPE-19 Working Cell Bank was planted onto T-175 flasks at a plant density of ~1e4 vc/cm$^2$ using 2 different growth media as listed below.

Medium #1: DMEM/F-12 + 10% FBS + 6 mM L-Glutamine

Medium #2: DMEM/F-12 + 10% FBS + 6 mM L-Glutamine + 10 ng/mL Epidermal Growth

Factor (EGF), 2.5 μM Dexamethasone, 1x essential amino acid cocktail, 1x GSEM (Sigma-Aldrich; L-alanine, L-asparagine, L-aspartic acid, L-glutamic acid, L-proline, L-serine, adenosine, guanosine, cytidine, uridine, and thymidine), and 1x Trace Elements A (Corning; Cupric Sulfate, Ferric Citrate, Sodium Selenite, and Zinc Sulfate).

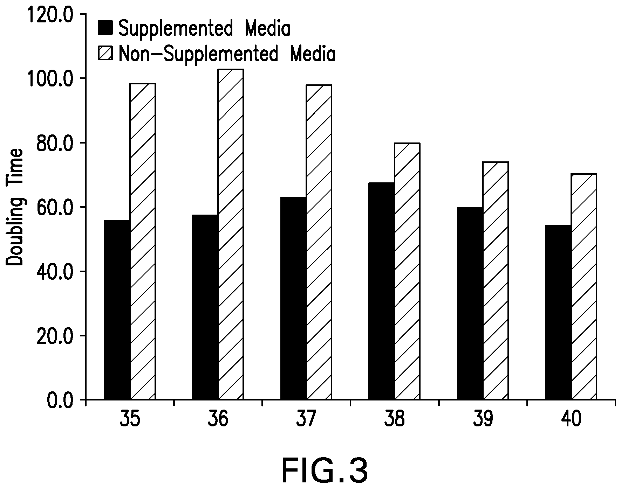

One flask from each medium was harvested daily for 7 days. Cells were passaged in the medium once they reach the targeted cell density of 8e4 vc/cm$^2$ or 6.67e7 cells/mL based on the daily cell counts (instrument used: Cedex cell counter). Cells in medium #1 were passaged 6 times and cells in medium #2 were passaged 8 times. Growth rate and doubling time were calculated. Cells grown in medium #2 (supplemented medium) had higher growth rate and lower doubling time in the first 3 passages compared to medium #1 (non-supplemented medium). See Figure 3.

The supplements added in combination specified above boost ARPE-19 cell growth in the presence of 10%FBS.

Another study was performed to investigate the effect of certain supplements on the growth of ARPE-19 cells with a lower amount of FBS (2%). The study was performed on T-flasks as described above.

ARPE-19 Working Cell Bank was planted onto T-175 flasks at plant density of ~1e4 vc/cm2 using 3 different growth media listed below.

Medium #3: DMEM/F-12 + 10% FBS + 6 mM L-Glutamine

Medium #4: DMEM/F-12 + 2% FBS + 6 mM L-Glutamine

## Medium #5: DMEM/F-12 + 2% FBS + 6 mM L-Glutamine + 10 ng/mL Epidermal Growth

Factor (EGF), 2.5 $\mu$M Dexamethasone, 1x essential amino acid cocktail, 1x Linoleic and Oleic acid mixture (Sigma-Aldrich).

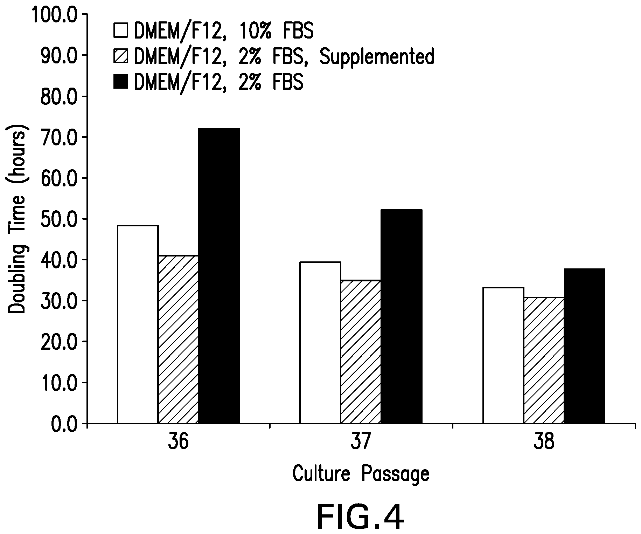

Cells were passaged in the media specified above for 3 passages under the same passaging schedule (7 day-6 day-4 day). The plant density for each passage was 1E4 vc/cm$^2$. The harvest density from each passage was measured by vicell cell counter. The average doubling time was calculated from the harvest density and plant density. The results are shown in Figure 4.

Surprisingly, cells passaged in medium #5 had equivalent doubling time to cells passaged in medium #3 despite medium #5 having lower FBS concentration than in medium #3. This shows that FBS concentration could be reduced but that adding certain supplements could provide an equivalent or superior effect on cell culture. In passage 36 and 37, the doubling time in medium #5 is also significantly lower than the doubling time in medium #4 which indicated the positive effect of that the supplements had on ARPE-19 cell growth.

These results indicate that the combination of EGF and dexamethasone, with different combinations of amino acids, was able to reduce doubling time of the ARPE-19 cells grown in media containing 2% FBS compared to ARPE-19 cells grown in media containing 10% FBS.

EXAMPLE 3: Demonstration of Improved ARPE-19 cell growth with addition of select supplements at various sera types

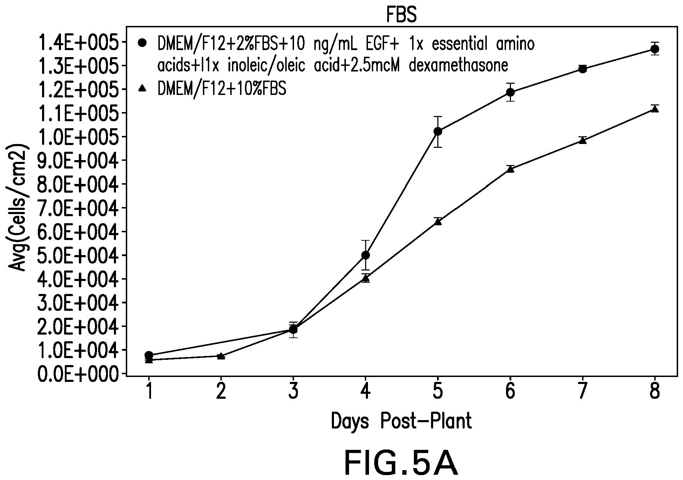

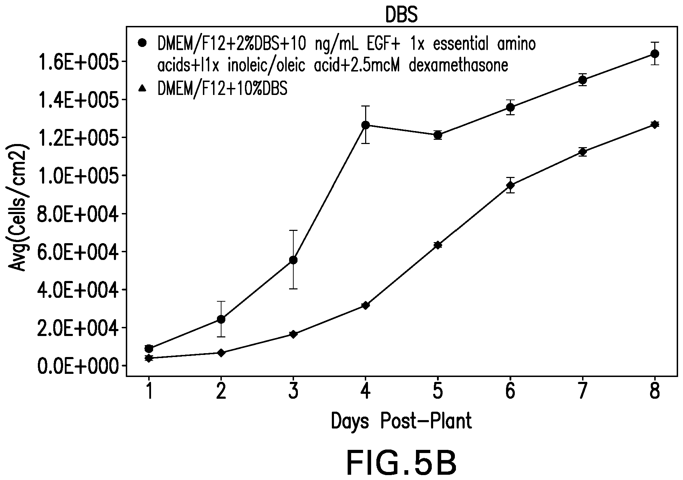

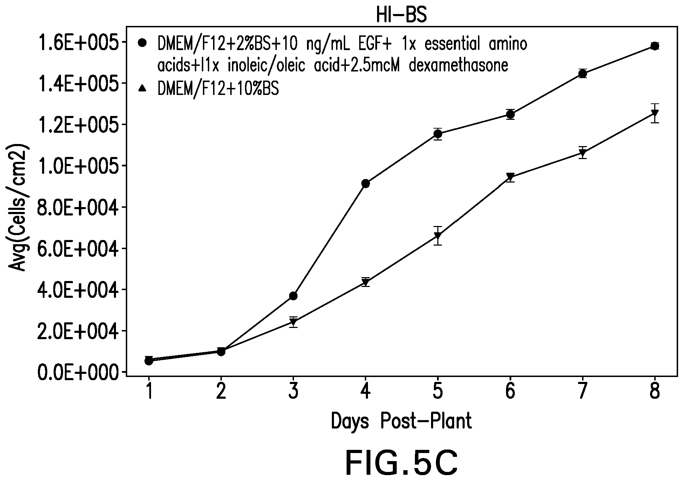

Various supplements were investigated in cell culture medium containing either fetabl bovine serum (FBS), bovine serum (BS) for the purpose of boosting ARPE-19 cell growth concentration. This investigation was performed on 96-well format. Cells were seeded at 1E4 vc/cm$^2$ (viable cells/cm$^2$) and growth was monitored by staining the cells on day 2, 3, 4, 5, 6, 7, and 8 post plant with cell nuclear stain (Hoechst) and quantifying the nuclear stain on an imager to generate cell growth data for each day. The cell growth data was analyzed statistically to assess the impact of the supplement on cell growth. The results are shown in Figures 5A-C.

The addition of supplements (EGF, essential amino acid, linoleic/oleic acid, and dexamethasone) to the DMEM/F12 media with 2%FBS resulted in 40% higher growth compared to 10%FBS alone. Similar observations were made when we tested the supplements in the presence of donor bovine serum (DBS) and Heat-Inactivated Bovine serum (BS).

EXAMPLE 4: Demonstration of Improved ARPE-19 Cell Growth in the presence of Select Supplements in a Microcarrier System inside Bioreactor

The effect of certain supplements was also tested in a microcarrier system inside bioreactors. Cells were planted on Cytodex™-1 microcarrier inside bioreactors with 2 different growth media. The cell growth in the bioreactors was measured using nucleocounter cell counting method. The average doubling time of the cells in the growth phase was calculated.

## Medium #6: DMEM/F-12 + 10% FBS + 6 mM L-Glutamine

Medium #7 : DMEM/F-12 + 10%FBS + 6 mM L-Glutamine+ 2.5 $\mu$M Dexamethasone, 10 ng/mL EGF, 1x essential amino acid + 1x GSEM + 1x Trace-Element A (Trace Elements A includes Cupric Sulfate, Ferric Citrate, Sodium Selenite, and Zinc Sulfate).

Table 7: ARPE-19 Population Doubling Time in Microcarrier inside a Bioreactor

| Growth Medium | Average Doubling Time (h) |
|---|---|
| DMEM/F 12 + 10% FBS+6 mM Glutamine | 47.9 |
| DMEM/F 12 + 10% FBS+ 6 mM Glutamine + Select supplements | 23.5 |

Based on the data presented above, an improved cell culture media will include include the following supplements to enhance ARPE-19 cell growth in the microcarrier system.

| Supplement Name | Concentration in Growth Medium | Unit |
|---|---|---|
| Dexamethanasone | 2.5 | $\mu$M |
| EGF | 10 | ng/mL |
| Essential amino acid | 1 | X |
| GSEM supplement | 1 | X |
| Trace Element-A | 1 | X |

EXAMPLE 5: Demonstration of Improved CMV Production in the presence of Select Supplements in Microcarrier System inside Bioreactor

The impact of the certain supplements on virus production was also assessed. Cells were planted on Cytodex™-1 microcarrier inside bioreactors with either DMEM/F12 + 10% FBS + 6 mM L-Glutamine growth medium or DMEM/F12 + 10% FBS + L-Glutamine + supplements. These bioreactors were then media-exchanged into infection/production medium containing DMEM/F12 + 1% FBS+ 5 $\mu$M Shield-1 or DMEM/F12+ 1% FBS + 5 $\mu$M Shield-1 + supplements. The specific components of the various growth media is provided in Table 8. The bioreactors were then infected with sufficient amount of virus seed and virus production was monitored over 15 days using Apogee flow cytometry (total particles/mL) and IRVE (infectivity).

Table 8:

| Condition | Growth Medium | Infection/Production Medium |
|---|---|---|
| 1 | DMEM/F12 + 10% FBS + 6 mM L-Glutamine | DMEM/F-12 + 1% FBS + 6 mM L-Glutamine + 5 $\mu$M Shield-1 |
| 2 | DMEM/F12 + 10% FBS+ 6 mM L-Glutamine | DMEM/F-12 + 1% FBS + 6 mM L-Glutamine + 5 $\mu$M Shield-1 + 2.5 $\mu$M Dexamethasone + 1x essential amino acid |
| 3 | DMEM/F-12 + 10% FBS + 6 mM L-Glutamine + 2.5 $\mu$M Dexamethasone, 10 ng/mL EGF, 1x essential amino acid + 1x GSEM + 1x Trace-Element A | DMEM/F-12 + 1% FBS + 6 mM L-Glutamine + 5 $\mu$M Shield-1 + 2.5 $\mu$M Dexamethasone, 10 ng/mL EGF, 1x essential amino acid + 1x GSEM + 1x Trace-Element A |

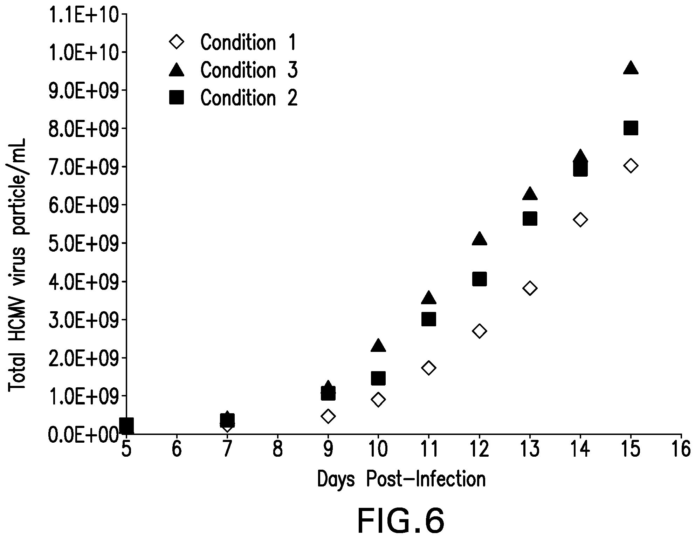

The volumetric production of virus as measured by Apogee flow cytometry is shown in Figure 6. Condition #3 yielded 35% higher total particle/mL compared to Condition #1 and approximately 20% higher total particle/mL compared to Condition #2.

Other embodiments are within the following claims.

## Claims

1. A cell culture medium comprising a basal cell culture medium that comprises serum and is further supplemented with between about 0.1 $\mu$M and about 10 $\mu$M of dexamethasone, between about 0.1 ng/mL and about 100 ng/mL of epidermal growth factor (EGF), 1X MEM essential amino acids, 1X-4X glutamine synthetase expression medium (GSEM) supplement and trace elements.

2. The medium of claim 1, wherein the basal cell culture medium is DMEM/F-12.

3. The medium of claim 1 or 2, wherein said serum is fetal bovine serum (FBS).

4. The medium of claim 3, wherein the FBS is present at a concentration between about 2% and about 10%, inclusive.

5. The medium of any one of claims 1-4, wherein the dexamethasone is present at a concentration between about 1

and about 10 μM.

6. The medium of any one of claims 1-5, wherein the EGF is present at a concentration between about 1 and about 40 ng/mL.

7. The medium of any one of claims 1 to 6, wherein the 1X MEM essential amino acids consist of arginine, cysteine, histidine, isoleucine, leucine, lysine, methionine, phenylalanine, threonine, tryptophan, tyrosine, and valine.

8. The medium of any one of claims 1 to 7, wherein the 1X GSEM supplement consists of a mixture of L-alanine, L-asparagine, L-aspartic acid, L-glutamic acid, L-proline, L-serine, adenosine, guanosine, cytidine, uridine, and thymidine.

9. The medium of any one of claims 1 to 8, wherein the trace elements are selected from cupric sulfate, ferric citrate, sodium selenite, and zinc sulfate.

10. A composition comprising the medium of any one of claims 1 to 9 and cells selected from ARPE-19, Vero, MDCK, MRC-5, BHK, CEM, and LL-I cells.

11. The composition of claim 10, wherein said composition further comprises a virus.

12. The composition of claim 11, wherein said virus is a human herpesvirus.

13. The composition of claim 12, wherein said human herpesvirus is human cytomegalovirus (HCMV).

14. The composition of claim 13, wherein said HCMV is a conditional replication defective HCMV mutant having a genomic sequence of SEQ ID NO: 1.

15. A method of increasing virus production in cells, said method comprising culturing the composition of any of claims 10 to 14, wherein said composition provides increased production of the virus by at least 10% relative to the production of virus grown in a composition that does not contain the supplements of claim 1.

**Patentansprüche**

1. Ein Zellkulturmedium, umfassend ein Basalzellkulturmedium, das Serum umfasst und ferner supplementiert ist mit zwischen etwa 0,1 μM und etwa 10 μM Dexamethason, zwischen etwa 0,1 ng/ml und etwa 100 ng/ml epidermalem Wachstumsfaktor (EGF), 1X MEM essentiellen Aminosäuren, 1X-4X Glutamin-Synthetase-Expressionsmedium (GSEM)-Supplement und Spurenelementen.

2. Das Medium nach Anspruch 1, wobei das Basalzellkulturmedium DMEM/F-12 ist.

3. Das Medium nach Anspruch 1 oder 2, wobei das Serum fetales Kälberserum (FKS) ist.

4. Das Medium nach Anspruch 3, wobei das FKS in einer Konzentration zwischen etwa einschließlich 2% und etwa einschließlich 10% vorliegt.

5. Das Medium nach einem der Ansprüche 1-4, wobei das Dexamethason in einer Konzentration zwischen etwa 1 und etwa 10 μM vorliegt.

6. Das Medium nach einem der Ansprüche 1-5, wobei der EGF in einer Konzentration zwischen etwa 1 und etwa 40 ng/ml vorliegt.

7. Das Medium nach einem der Ansprüche 1 bis 6, wobei die 1X MEM essentiellen Aminosäuren aus Arginin, Cystein, Histidin, Isoleucin, Leucin, Lysin, Methionin, Phenylalanin, Threonin, Tryptophan, Tyrosin und Valin bestehen.

8. Das Medium nach einem der Ansprüche 1 bis 7, wobei das 1X GSEM-Supplement aus einer Mischung aus L-Alanin, L-Asparagin, L-Asparaginsäure, L-Glutaminsäure, L-Prolin, L-Serin, Adenosin, Guanosin, Cytidin, Uridin und Thymidin besteht.

9. Das Medium nach einem der Ansprüche 1 bis 8, wobei die Spurenelemente ausgewählt sind aus Kupfersulfat, Eisen(III)-citrat, Natriumselenit und Zinksulfat.

10. Eine Zusammensetzung, die das Medium nach einem der Ansprüche 1 bis 9 und Zellen, ausgewählt aus ARPE-19-, Vero-, MDCK-, MRC-5-, BHK-, CEM- und LL-I-Zellen, umfasst.

11. Die Zusammensetzung nach Anspruch 10, wobei die Zusammensetzung ferner ein Virus umfasst.

12. Die Zusammensetzung nach Anspruch 11, wobei das Virus ein humanes Herpesvirus ist.

13. Die Zusammensetzung nach Anspruch 12, wobei das humane Herpesvirus humanes Cytomegalievirus (HCMV) ist.

14. Die Zusammensetzung nach Anspruch 13, wobei das HCMV eine konditionale replikationsdefekte HCMV-Mutante mit einer Genomsequenz von SEQ ID NO: 1 ist.

15. Ein Verfahren zur Steigerung der Virusproduktion in Zellen, wobei das Verfahren das Kultivieren der Zusammensetzung nach einem der Ansprüche 10 bis 14 umfasst, wobei die Zusammensetzung eine um wenigstens 10% gesteigerte Produktion des Virus ergibt, verglichen mit der Produktion von in einer Zusammensetzung, die die Supplemente nach Anspruch 1 nicht enthält, angezüchtetem Virus.

## Revendications

1. Milieu de culture cellulaire comprenant un milieu de culture cellulaire basal qui comprend du sérum et est en outre enrichi par environ 0,1 $\mu$M à environ 10 $\mu$M de dexaméthasone, environ 0,1 ng/mL à environ 100 ng/mL de facteur de croissance épidermique (EGF), des acides aminés essentiels de MEM 1X, du supplément de milieu d'expression de la glutamine synthétase (GSEM) 1X-4X et des oligo-éléments.

2. Milieu selon la revendication 1, dans lequel le milieu de culture de cellules basales est le DMEM/F-12.

3. Milieu selon les revendications 1 ou 2, dans lequel ledit sérum est du sérum bovin foetal (FBS).

4. Milieu selon la revendication 3, dans lequel le FBS est présent en une concentration comprise entre environ 2 % et environ 10 %, inclus.

5. Milieu selon l'une quelconque des revendications 1 à 4, dans lequel la dexaméthasone est présente en une concentration comprise entre environ 1 et environ 10 $\mu$M.

6. Milieu selon l'une quelconque des revendications 1 à 5, dans lequel l'EGF est présent en une concentration comprise entre environ 1 et environ 40 ng/mL.

7. Milieu selon l'une quelconque des revendications 1 à 6, dans lequel les acides aminés essentiels de MEM 1X sont constitués de l'arginine, la cystéine, l'histidine, l'isoleucine, la leucine, la lysine, la méthionine, la phénylalanine, la thréonine, le tryptophane, la tyrosine et la valine.

8. Milieu selon l'une quelconque des revendications 1 à 7, dans lequel le supplément de GSEM 1X est constitué d'un mélange de L-alanine, L-asparagine, acide L-aspartique, acide L-glutamique, L-proline, L-sérine, adénosine, guanosine, cytidine, uridine et thymidine.

9. Milieu selon l'une quelconque des revendications 1 à 8, dans lequel les oligo-éléments sont choisis parmi le sulfate de cuivre, le citrate ferrique, le sélénite de sodium et le sulfate de zinc.

10. Composition comprenant le milieu selon l'une quelconque des revendications 1 à 9 et des cellules sélectionnées parmi des cellules ARPE-19, Vero, MDCK, MRC-5, BHK, CEM et LL-I.

11. Composition selon la revendication 10, ladite composition comprenant en outre un virus.

12. Composition selon la revendication 11, dans laquelle ledit virus est un herpèsvirus humain.

**13.** Composition selon la revendication 12, dans laquelle ledit herpèsvirus humain est le cytomégalovirus humain (HCMV).

**14.** Composition selon la revendication 13, dans laquelle ledit HCMV est un mutant HCMV conditionnel défectif pour la réplication présentant une séquence génomique de la SEQ ID NO :1.

**15.** Procédé permettant d'augmenter la production virale dans des cellules, ledit procédé comprenant la mise en culture de la composition selon l'une quelconque des revendications 10 à 14, dans lequel ladite composition fournit une production accrue du virus d'au moins 10 % par rapport à une production de virus cultivé dans une composition ne contenant pas les suppléments selon la revendication 1.

| Sorted Parameter Estimates | | | | | |
|---|---|---|---|---|---|
| Term | Estimate | Std Error | t Ratio | | Prob>\|t\| |
| EGF(0,10) | 141240.22 | 31376.09 | 4.50 | | 0.0041* |
| Non−essential amino acids(0,1) | −111823.3 | 31060.74 | −3.60 | | 0.0114* |
| Essential Amino Acids(0,1) | 99665.072 | 31060.74 | 3.21 | | 0.0184* |
| Linoleic and Oleic acid(0,1) | 72356.739 | 31060.74 | 2.33 | | 0.0587 |
| Glutathione(0,0.02) | 47485.072 | 31060.74 | 1.53 | | 0.1772 |
| Insulin(0,10) | −9618.116 | 31376.09 | −0.31 | | 0.7696 |
| Dexamethasone(0,1) | −4498.406 | 31060.74 | −0.14 | | 0.8896 |
| Hydrocortisone(0,10) | −734.7826 | 31376.09 | −0.02 | | 0.9821 |

# FIG.1

FIG.2

FIG.3

FIG.4

FIG.5A

FIG.5B

FIG.5C

FIG.6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 9546355 B
- US 6656719 B
- JP 2006158388 B
- US 9249427 B
- US 20160354461
- WO 1998010311 A1
- US 20090215169
- US 62661928

### Non-patent literature cited in the description

- **CROUGH et al.** *Clinical Microbiology Reviews,* 2009, vol. 22, 76-98
- Cytomegalovirus. **MOCARSKI et al.** Field Virology. 2007, 2701-2772
- **ADLER.** *J. Clin Virol,* 2008, vol. 41, 231
- **ARVIN et al.** *Clin Infect Dis,* 2004, vol. 39, 233
- **REVELLO et al.** *J Med Virol,* 2008, vol. 80, 1415
- **WANG et al.** *Sci Transl Med,* 2016, vol. 8, 362ra145
- **COSTA et al.** *Nature,* 1974, vol. 252, 745-746
- **SOLODUSHKO et al.** *Am J Physiol Lung Cell Mol Physio,* 2009, vol. 297, L538-45
- **TANAKA et al.** *J Gen Virol.,* 1984, vol. 65, 1759-67
- **TANAKA et al.** *Virology,* 1984, vol. 136, 448-52
- **ILOTT et al.** *Epidemiol Infect,* 1997, vol. 118, 181-7
- **LINDL, T.** Zell- und Gewebekultur. Spektrum Akademischer Verlag, 2002
- **DOYLE et al.** *Cell and Tissue Culture: Laboratory Procedures,* 1999, vol. 27D (3), 3
- Tissue Culture. Academic Press, 1973
- **FRESHNEY.** Culture of animal cells: A manual of basic technique. Wiley-Liss Inc, 2000
- **BRITT.** Human Cytomegalovirus: Propagation, Quantification, and Storage. *Current Protocols in Microbiology,* 2010, vol. 18
- **CHAMBERS et al.** *Appl Microbiol,* 1971, vol. 22, 914-8
- **SCHNEIDER-OHRUM et al.** *J Virol,* 2016, vol. 90, 10133-10144
- **SCHNEIDER-OHRUM et al.** *J Virol.,* 2016, vol. 90, 10133-10144
- **LILJA et al.** *Vaccine,* 2012, vol. 30, 6980
- **MARZI et al.** *Sci Rep,* 2016, vol. 6, 21674
- **TIERNEY et al.** *Vaccine,* 2012, vol. 30, 3047
- **CLACKSON et al.** *Proc Natl Acad Sci USA,* 1998, vol. 95, 10437-42
- **WANG ; SHENK.** *Proc Natl Acad Sci USA,* 2005, vol. 102, 1815
- **RYCKMAN et al.** *J. Virol.,* 2008, vol. 82, 60